# EUROPEAN PATENT SPECIFICATION

(11) **EP 2 283 769 B1**
(45) Date of publication and mention of the grant of the patent: **14.06.2017**
(21) Application number: 10172266.8
(22) Date of filing: 09.08.2010
(51) Int. Cl.: A61B 1/04

(54) **Endoscopic device**
Endoskopvorrichtung
Dispositif endoscopique

(30) Priority: 10.08.2009 JP 2009185687
(43) Date of publication of application: 16.02.2011
(60) Divisional application: 17169750.1
(73) Proprietor: Fujifilm Corporation, Minato-ku Tokyo (JP)
(72) Inventor: Endo, Azuchi, Kanagawa (JP); Erikawa, Akihiko, Kanagawa (JP); Ozawa, Satoshi, Kanagawa (JP); Mizuyoshi, Akira, Kanagawa (JP)
(74) Representative: Klunker . Schmitt-Nilson . Hirsch

(56) References cited:
- EP-A1- 1 795 798
- EP-A1- 2 130 484

## Description

### BACKGROUND OF INVENTION

### Field of the Invention

The present invention relates to an endoscopic device.

### Description of the Related Art

A white light source is generally used as illuminating light of an endoscopic device, and an endoscopic device including a light source for highlighting a state of a mucosal tissue through illumination with light of a specific narrowed band wavelength or for observation with special light such as observation of self-fluorescence from a fluorescent substance precedently administered is recently put to practical use (JP-B-3583731, JP-B-6-40174). When such an endoscopic device is employed, biological information that cannot be obtained from general observation images, such as a neovascular fine structure produced in a mucosal layer or a submucosal layer, or a highlighted lesion, may be easily visualized.

Furthermore, the illuminating light of an endoscopic device is desired to be set to a color tone suitable to diagnosis because a color tone of a light source proper to be employed for finding a lesion differs depending upon a site to be observed. For example, in diagnosis of the large intestine, a lesion may be more definitely identified by observing the form and the color of the inside of the large intestine in observation with low magnifying power and observing blood vessels of a surface layer in observation with high magnifying power. In the endoscopic device, however, although the illuminating light may be switched between white light for general observation and special light for observation with special light, the color tone of the illuminating light cannot be arbitrarily changed in accordance with an observation target or in accordance with various diagnosis situations such as enlargement observation.

### SUMMARY OF INVENTION

An object of the invention is providing an endoscopic device capable of arbitrarily changing a color tone of illuminating light in accordance with an observation target or a diagnosis situation.

The present invention provides an endoscopic device having the features of claim 1.

In accordance with the preamble of claim 1, EP-A-1 795 798 discloses an endoscopic device comprising a light emitting structure which includes an excitation light source and a wavelength conversion member which absorbs excitation light and emits light having a converted wavelength. Plural excitation light sources may be provided.

According to the endoscopic device of the invention, a color tone of illuminating light may be arbitrarily changed in accordance with an observation target or a diagnosis situation.

### BRIEF DESCRIPTION OF THE DRAWINGS

FIG. 1 is a block diagram illustrating the structure of an endoscopic device and corresponding to a diagram for explaining an embodiment of the invention;
FIG. 2 is a diagram illustrating an example of the external structure of the endoscopic device of FIG. 1;
FIG. 3 is a graph illustrating emission spectra of a violet laser beam emitted from a violet laser light source, a blue laser beam emitted from a blue laser light source and light obtained after wavelength conversion, by a phosphor, of the blue laser beam;
FIG. 4 is a diagram illustrating an example of the structure of an objective lens unit;
FIG. 5 is a block diagram illustrating the structure of an optical system of the objective lens unit;
FIG. 6 is a graph illustrating the relationship between observation magnifying power and a light quantity ratio;
FIG. 7 is an explanatory diagram schematically illustrating blood vessels of a mucosal surface layer of a biological tissue;
FIG. 8 is an explanatory diagram roughly illustrating examples of display of observation images obtained by the endoscopic device;
FIG. 9 is a graph illustrating the relationship between an applied current and an emitted light quantity;
FIG. 10 is a graph illustrating a pulse current superposed waveform of the applied current;
FIG. 11 is an explanatory diagram illustrating various drive waveforms (a), (b) and (c) generated through pulse-modulated control;
FIG. 12 is a graph illustrating exemplary control in which emitted light quantities of light sources are alternately maximized;
FIG. 13 is a graph illustrating the relationship between a distance between a region to be observed and an endoscope tip portion and a received light quantity;
FIG. 14 is a block diagram roughly illustrating the structure of an endoscopic device in which a color tone of illuminating light is changed in accordance with a site of a region to be observed;
FIG. 15 is an explanatory diagram illustrating test order information including patient information and test information;
FIG. 16 is a block diagram roughly illustrating the structure of an endoscopic device in which a color tone of illuminating light is changed in accordance with the type of an endoscope;
FIG. 17 is an explanatory diagram illustrating an example of a registration table of individual identification information including the type name of an endoscope;
FIG. 18 is an explanatory diagram illustrating an example of a registration table of individual identification information including the type and individual specificity of a phosphor;
FIG. 19 is a diagram roughly illustrating the structure of an endoscopic device in which one optical fiber is used as an optical path extending from a light source device to an endoscope;
FIG. 20 is a graph illustrating emission spectra of illuminating light obtained by the light source device of FIG. 19 and a phosphor;
FIG. 21 is a diagram roughly illustrating the structure of another exemplary endoscopic device using a large number of laser light sources;
FIG. 22 is a diagram roughly illustrating the structure of another exemplary endoscopic device using a white light source and a laser light source; and
FIG. 23 is a diagram roughly illustrating the structure of an endoscopic device including a light emitting diode provided in a tip portion of an endoscope.

### DESCRIPTION OF EXEMPLARY EMBODIMENTS

Now, an embodiment of the invention will be described in detail with reference to the accompanying drawings.

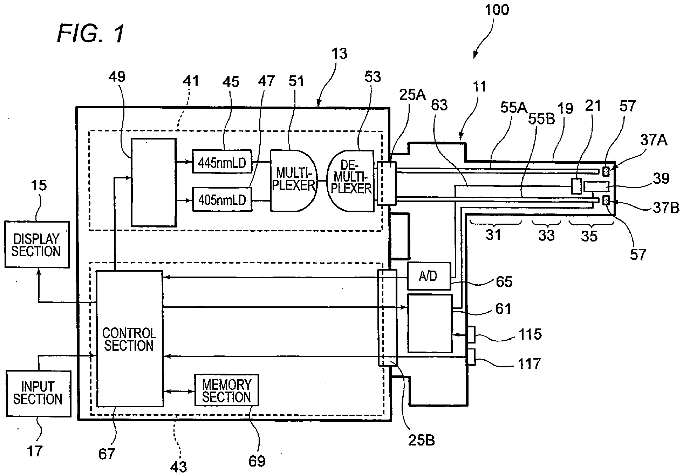

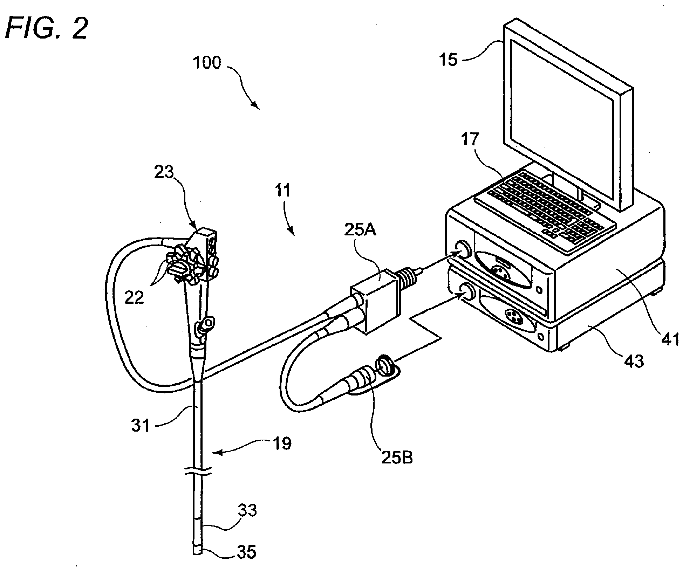

FIG. 1 is a block diagram conceptually illustrating the structure of an endoscopic device used for explaining the embodiment of the invention, and FIG. 2 is a diagram illustrating an exemplary external structure of the endoscopic device of FIG. 1.

The endoscopic device 100 includes an endoscope 11 and a controller 13 connected to the endoscope 11. The controller 13 is connected to a display section 15 for displaying image information and the like and an input section 17 for accepting an input operation. The endoscope 11 is an electronic endoscope including an illuminating light optical system for emitting illuminating light from a tip of an endoscope insert section 19 and an imaging optical system including an imaging device 21 for obtaining a pickup image of a region to be observed.

As illustrated in FIGS. 1 and 2, the endoscope 11 includes the endoscope insert section 19 to be inserted into an observed body, an operation section 23 for operating to bend the tip of the endoscope insert section 19 to observe, and connector sections 25A and 25B for removably connecting the endoscope 11 to the controller 13. Although not shown in the drawings, various channels such as a forceps channel for inserting a tissue collecting tool or the like and a channel for supplying air or water are provided inside the endoscope 11.

The endoscope insert section 19 includes a flexible soft portion 31, a bending portion 33 and a tip portion (hereinafter referred to also as the endoscope tip portion) 35. In the endoscope tip portion 35, radiation ports 37A and 37B through which a region to be observed is radiated with light and the imaging device 21 for obtaining image information of the region to be observed, such as a CCD (Charge Coupled Device) image sensor or a CMOS (Complementary Metal-Oxide Semiconductor) image sensor, are provided. It is noted that the imaging device 21 is provided with an objective lens unit 39 having a zooming function for controlling a focal distance and an automatic focusing function for focusing on a subject.

The bending portion 33 is disposed between the soft portion 31 and the tip portion 35 and is bendable through a rotation operation of an angle knob 22 disposed on the operation section 23 or a function of an actuator. This bending portion 33 may be bent at an arbitrary angle in an arbitrary direction in accordance with a site or the like of a subject of the endoscope 11, so that the radiation ports 37A and 37B of the endoscope tip portion 35 and the observation direction of the imaging device 21 may be made to aim at a desired observation site. Furthermore, although not shown in the drawings, the radiation ports 37A and 37B of the endoscope insert portion 19 are provided with cover glasses or lenses.

The controller 13 includes a light source device 41 for generating illuminating light to be supplied to the radiation ports 37A and 38B of the endoscope tip portion 35 and a processor 43 for image processing image signals supplied from the imaging device 21, and is connected to the endoscope 11 through the connector sections 25A and 25B. Also, the processor 43 is connected to the display section 15 and the input section 17 as described above. The processor 43 performs image processing on image signals transmitted from the endoscope 11 on the basis of an instruction given through the operation section 23 of the endoscope 11 or the input section 17, so as to generate a display image and supply the generated image to the display section 15.

The light source device 41 includes, as light sources, a blue laser light source (i.e., a first light source) 45 with a center wavelength of 445 nm and a violet laser light source (i.e., a second light source) 47 with a center wavelength of 405 nm. Light emitted from semiconductor light emitting diodes of these light sources 45 and 47 is individually controlled by a light source control section 49, so that a light quantity ratio between output light of the blue laser light source 45 and output light of the violet laser light source 47 may be changed. In other words, the light source control section 49 functions as color tone controlling means for the illuminating light.

As each of the blue laser light source 45 and the violet laser light source 47, a broad-area type InGaN-based laser diode may be used, and alternatively, an InGaNAs-based laser diode or a GaNAs-based laser diode may be used. Alternatively, a luminous element such as a light emitting diode may be used as the light source.

Laser beams emitted from the light sources 45 and 47 are input to an optical fiber by a condensing lens (not shown) and transmitted to the connector section 25A through a combiner 51, that is, a multiplexer, and a coupler 53, that is, a demultiplexer. It is noted that this structure does not limit the invention but the laser beams emitted from the light sources 45 and 47 may be directly sent to the connector section 25A without using the combiner 51 and the coupler 53.

The laser beams supplied to the connector section 25A and obtained by multiplexing the blue laser beams of the center wavelength of 445 nm and the violet laser beams of the center wavelength of 405 nm are propagated to the endoscope tip portion 35 of the endoscope 11 through optical fibers 55A and 55B. The blue laser beams excite a phosphor 57 corresponding to a wavelength conversion member disposed at the light outputting end of each of the optical fibers 55A and 55B of the endoscope tip portion 35, so as to cause fluorescence emission. Also, part of the blue laser beams directly passes through the phosphor 57. The violet laser beams pass through the phosphor 57 without exciting it, so as to work as illuminating light of a narrowed band wavelength.

Each of the optical fibers 55A and 55B is a multimode fiber, and for example, a thin cable with a core diameter of 105 µm, a cladding diameter of 125 µm and a diameter including a protective layer working as a casing of 0.3 through 0.5 mm may be used.

The phosphor 57 includes a plurality of kinds of phosphors that absorb part of the blue laser beams for emitting green-to-yellow light when excited (such as a YAG-based phosphor and a phosphor including BAM (BaMgAl₁₀O₁₇) or the like). Thus, the green to yellow light emitted through excitation under radiation of the blue laser beams and the part of the blue laser beams not absorbed but transmitted by the phosphor 57 are multiplexed, so as to give white (pseudo white) illuminating light. When a semiconductor light emitting diode is used as the excitation light source as in this exemplary structure, white light with high intensity may be obtained with high luminous efficacy, and the intensity of the white light may be easily controlled. In addition, change in the color temperature and the chromaticity of the white light may be suppressed to be small.

The phosphor 57 and a light deflecting/diffusing member 59 may prevent phenomenon such as noise superposition obstructing imaging derived from speckle caused due to coherence of laser beams or occurrence of flicker in displaying dynamic images. Furthermore, in consideration of a difference in refractive index between a fluorescent material and a fixing/setting resin working as a filler included in the phosphor, the phosphor 57 is preferably made of a material having such particle sizes of the fluorescent material and the filler that light of the infrared region is little absorbed and largely diffused. Thus, the diffusion effect for light of red or the infrared region may be increased without lowering the light intensity, and there is no need to provide optical path changing means such as a concave lens and the optical loss may be reduced.

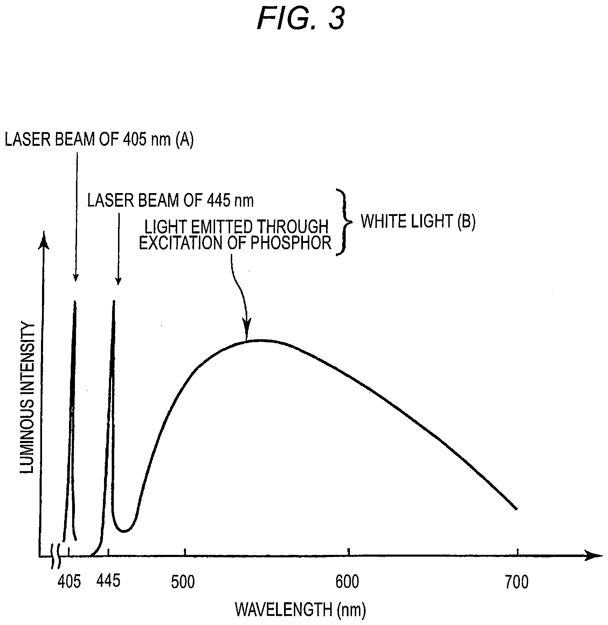

FIG. 3 is a graph illustrating emission spectra of the violet laser beam emitted from the violet laser light source 47, the blue laser beam emitted from the blue laser light source 45 and the light obtained through the wavelength conversion, by the phosphor 57, of the blue laser beam. The violet laser beam is expressed by an emission line with a center wavelength of 405 nm (i.e., a profile A). Also, the blue laser beam is expressed by an emission line with a center wavelength of 445 nm, and the light emitted through excitation from the phosphor 57 under radiation of the blue laser beam has a spectral intensity distribution in which the emission intensity is increased in a wavelength band of approximately 450 nm through 700 nm. The light emitted through the excitation and a profile B derived from the blue laser beam together generate the aforementioned white light.

At this point, white light herein does not strictly mean merely light including all wavelength components of visible light but may be light including light of specific wavelength bands such as R, G and B. For example, light including wavelength components from green to red or light including wavelength components from blue to green is also regarded as white light in a broad sense.

In this endoscopic device 100, the luminous intensities of the profile A and the profile B are controlled to be relatively increased/decreased by the light source control section 49 so as to generate illuminating light of an arbitrary color tone. Therefore, illuminating light with different characteristics may be obtained depending upon a mixing ratio between the profiles A and B.

Referring to FIG. 1 again, the illuminating light generated from the blue laser beams, the light emitted through the excitation of the phosphor 57 and the violet laser beams is radiated to the region to be observed of the subject from the tip portion 35 of the endoscope 11. Then, the state of the region to be observed radiated by the illuminating light is focused on the imaging device 21 by the objective lens unit 39 for obtaining a pickup image. The objective lens unit 39 is specified for the zooming magnifying power by a zoom control section 61 so as to obtain the focus with the specified zooming magnifying power.

An image signal of the pickup image output from the imaging device 21 after the imaging is transmitted to an A/D converter 65 through a scope cable 63 to be converted into a digital signal, and the digital signal is input to a control section 67 of the processor 43 through the connector section 25B. In the control section 67, the input digital image signal is converted into image data for performing appropriate image processing, so as to generate desired output image information. Then, the thus obtained image information is displayed on the display section 15 as an endoscope observation image, and is stored in a memory section 69 of a memory or a storage device if necessary.

The memory section 69 may be included in the processor 43 as illustrated in FIG. 1 or may be connected to the processor 43 through a network. It is noted that information of an endoscope observation image stored in the memory section 69 may be recorded in association with information of a light quantity ratio employed in obtaining the pickup image. In this manner, the stored endoscope observation image may be accurately read after the observation with the endoscope and appropriate image processing for, for example, standardizing the image may be performed in accordance with the light quantity ratio, and thus, the application range of the endoscope observation image may be expanded.

In the endoscopic device 100 having the aforementioned structure, the color tone of the illuminating light emitted from the endoscope tip portion 35 may be continuously changed. Furthermore, since the laser light source is used, the color tone may be controlled with high resolution, the color tone is minimally changed with time, and the light quantity may be controlled with high responsibility. Moreover, since the luminous efficacy is high, the power consumption may be reduced. In endoscopic diagnosis, a lesion may be easily found or diagnosed by setting the color tone of the illuminating light to a color tone suitable to the diagnosis, and therefore, it is significant for improvement of diagnostic accuracy to make the illuminating light changeable at arbitrary timing during the endoscopic diagnosis. Now, examples of the structure of the endoscopic device in which the color tone of the illuminating light is changed in accordance with any of various parameters will be successively described.

### <Structure in which the color tone of the illuminating light is changed in accordance with observation magnifying power for a region to be observed>

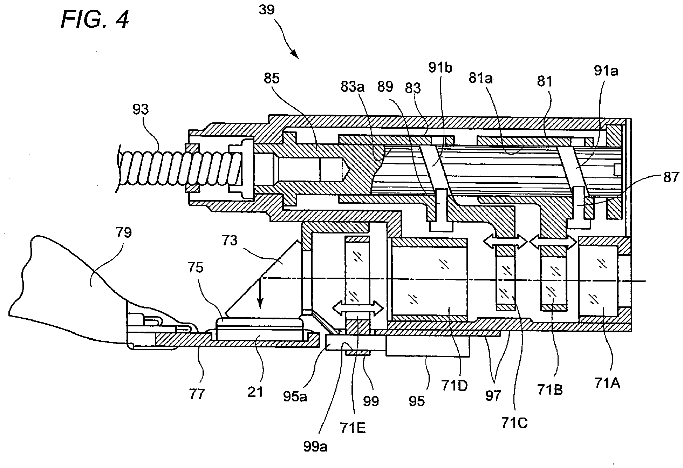

First, an exemplary structure in which the color tone is changed in accordance with a distance between a region to be observed and the endoscope tip portion will be described. An observation distance, that is, a distance between the region to be observed and the endoscope tip portion 35 (see FIG. 1), is set to be small in enlargement observation and to be large in observation with a large visual field, and therefore, as the observation magnifying power is higher, the observation distance is smaller. The observation magnifying power is arbitrarily set by an operator of the endoscope by using a zooming mechanism of the objective lens unit 39. At this point, an exemplary structure of the objective lens unit 39 is illustrated in FIG. 4.

As illustrated in FIG. 4, an optical system of the objective lens unit 39 includes a fixed lens 71 A, a first movable lens 71B and a second movable lens 71C for varying magnifying power constructed as varifocal lenses, a fixed lens 71D and a third movable lens 71E for focus adjustment arranged in this order from a side of an imaging target, and the imaging device 21 is disposed behind the third movable lens 71E with a prism 73 and a cover glass 75 disposed thereon. A signal obtained by the imaging device 21 is supplied to the processor 43 (see FIG. 1) through a circuit board 77 and a signal line 79.

The first movable lens 71B is held by a holding frame 81 having an engaging hole 81 a, the second movable lens 71C is held by a holding frame 83 having an engaging hole 83a, and the respective lenses 71B and 71C are attached on a cam shaft 85 in a cylindrical shape with the engaging holes 81a and 83a fit around the outer circumference of the cam shaft 83. Cam pins 87 and 89 are fit respectively in the engaging holes 81a and 83a to be protruded, and the cam shaft 85 is provided with cam grooves 91 a and 91b formed at different inclination angles against its shaft line, so that the cam pins 87 and 89 may be engaged respectively with the cam grooves 91 a and 91b.

The cam shaft 85 is linked with one end of a linear transmission member 93 of a coiled coil spring or the like, and the other end of the linear transmission member 93 is attached on a rotation shaft of a motor not shown provided in the operation section 23 (see FIG. 1). Accordingly, when the cam shaft 85 is rotated through the linear transmission member 93 by driving the motor, the first movable lens 71B and the second movable lens 71C are moved in the forward/backward direction along the optical axis by different distances owing to the engagement between the cam grooves 91a and 91b and the cam pins 87 and 89, resulting in attaining optical variable power (for enlargement) or the like. In other words, the first and second movable lenses 71B and 71C together construct a varifocal optical system, so as to attain the optical variable power by relatively moving backward/forward (namely, so as to vary an observation distance, an observation depth, a focal length and the like). This structure corresponds to a variable power driving mechanism.

On the other hand, a compact and high-speed actuator 95 utilizing a piezoelectric element is provided on a supporting section 97 for driving the third movable lens 71E for focus adjustment, and the periphery of a drive shaft 95a of the actuator 95 is movably fit in an engaging hole 99a of a holding frame 99 of the third movable lens 71E. In this actuator 95, the piezoelectric element is provided on the drive shaft 95a, so as to move the drive shaft 95a backward/forward at high and low speed by the piezoelectric element, thereby moving the third movable lens 71E backward/forward. This structure corresponds to a focus driving mechanism. It is noted that the actuator 95 may be another compact linear actuator such as an electrostatic actuator.

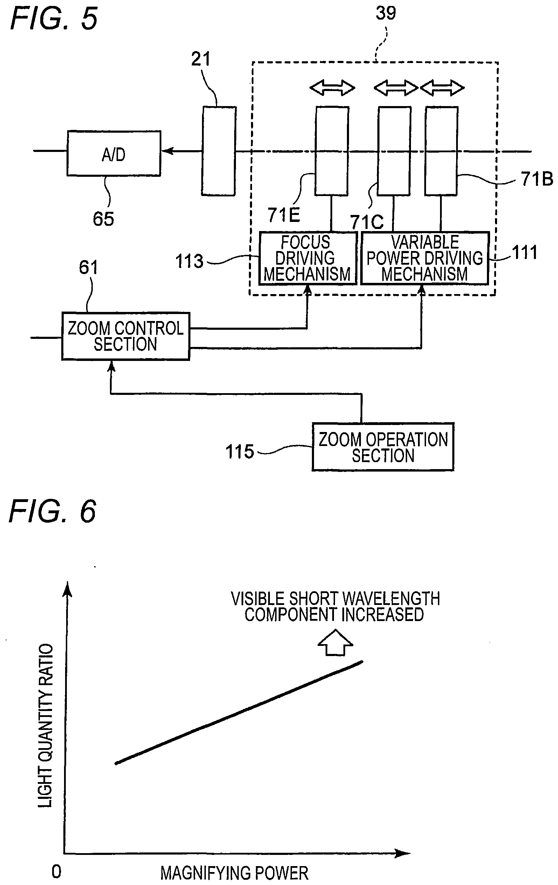

The optical system of the objective lens unit 39 is simply illustrated as a structure of a block diagram of FIG. 5. Specifically, the zoom control section 61 controls the variable power driving mechanism 111 and the focus driving mechanism 113 of the objective lens unit 39 so as to set desired magnifying power and adjust the focus with the magnifying power. The magnifying power is set by operating a zoom operation section 115 connected to the zoom control section 61. The zoom operation section 115 corresponds to operation buttons or slide switches arranged in the operation section 23 (see FIG. 2) and is constructed to be able to continuously change the observation magnifying power.

In endoscopic diagnosis of, for example, the large intestine, the form and the color of the surface of the mucosa may be observed with low magnifying power and blood vessels of the surface layer may be observed with high magnifying power, and hence, a preferable color tone of the illuminating light differs depending upon the observation magnifying power. Specifically, the illuminating light is preferably white when the magnifying power is low and the illuminating light preferably includes a visible short-wavelength component in a large ratio for extracting information of the blood vessel of the surface layer when the magnifying power is high.

Therefore, as illustrated as the relationship between the observation magnifying power and the light quantity ratio in FIG. 6, the color tone of the illuminating light is changed in accordance with the observation magnifying power. Specifically, an output light quantity ratio between the blue laser light source 45 and the violet laser light source 47 is controlled by the light source control section 49, so as to increase the output light quantity of the violet laser light source 47 as the observation magnifying power set by the zoom control section 61 is higher. As a result, in the light quantity ratio between the profile A with the short wavelength and the profile B of the white light illustrated in FIG. 3, the light quantity of the profile A is increased as the observation magnifying power is higher, so as to attain illuminating light including the short-wavelength component in a larger ratio. Since this change of the color tone is performed on a real-time basis simultaneously with the zoom operation performed by an operator of the endoscope or the like, the control may be carried out with high responsibility. It is noted that the relationship illustrated in FIG. 6 is not limited to the linear relationship but may be a non-linear curved relationship or a relationship changed in a stepwise manner.

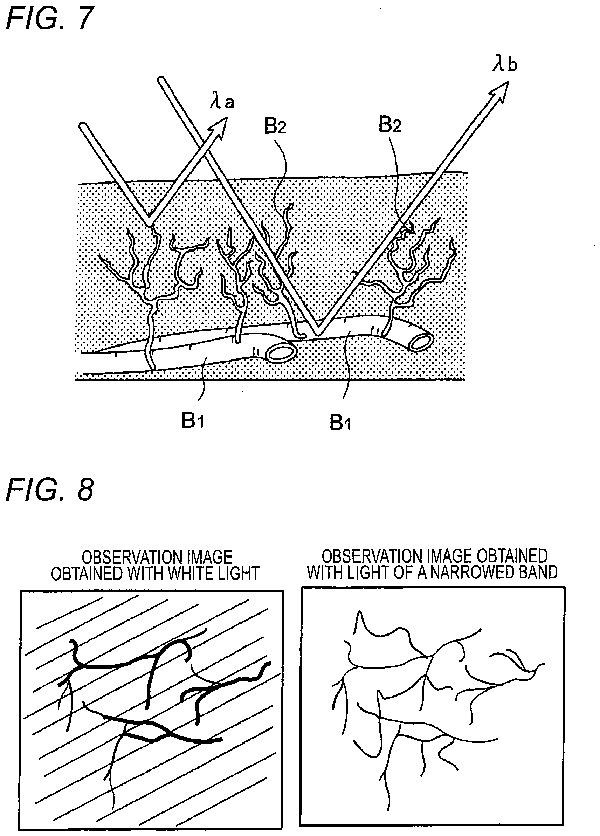

At this point, FIG. 7 is an explanatory diagram schematically illustrating blood vessels present in a mucosal surface layer of a biological tissue. In the mucosal surface layer of the biological tissue, blood capillaries B2 such as dendritic vasoganglion are formed from blood vessels B1 of a mucosal deep layer, and it has been reported that a lesion of the biological tissue appears in a fine structure of the blood capillary B2 or the like. Therefore, an attempt is being made for early detection of a small lesion or diagnosis of a lesion area by observing a highlighted image of the blood capillaries of the mucosal surface layer.

When the illuminating light enters a biological tissue, although the incident light propagates while spreading in the biological tissue, the absorbing/scattering characteristic of the biological tissue has dependency on wavelength, and hence, the scattering characteristic tends to be higher when the wavelength is shorter. In other words, the depth of invasion of the light varies depending upon the wavelength of the illuminating light. Therefore, when the illuminating light is in a wavelength region λa in the vicinity of 400 nm, blood vessel information of the blood capillaries of the mucosal surface layer may be obtained, and when it is in a wavelength region λb in the vicinity of 500 nm, blood vessel information including blood vessels of a deeper layer may be obtained. Accordingly, for the observation of blood vessels of the surface layer of the biological tissue, a light source with a center wavelength of 360 through 800 nm, preferably 365 through 515 nm and more preferably 400 nm through 470 nm is used.

As roughly illustrated in FIG. 8 as display examples of an observation image obtained by the endoscopic device, in an observation image obtained by using illuminating light of white light, an image of blood vessels of a comparatively deep mucosal layer is obtained while fine blood capillaries of a mucosal surface layer are blurred. On the other hand, in an observation image obtained by using illuminating light including the visible short-wavelength component in a large ratio, fine blood capillaries of the mucosal surface layer are clear.

In other words, in observation of blood vessels of a surface layer with high observation magnifying power, the color tone of the illuminating light is controlled to increase blueness, and in observation with low observation magnifying power, the illuminating light is controlled to be white with the blueness suppressed, and thus, the illuminating light suitable to the diagnosis performed by the operator of the endoscope may be attained. In this manner, in the adjustment of the observation magnifying power, there is no need for the operator to perform a complicated operation such as adjustment of the output light quantities of the blue laser light source 45 and the violet laser light source 47 but the illuminating light with a color tone optimum to an observation site may be always automatically attained.

Furthermore, in this endoscopic device 100, the change of the color tone of the illuminating light is performed by driving the light sources 45 and 47, and therefore, as compared with a case where the color tone is changed by using a color filter or the like, the light utilization may be improved and noise caused in an observation image may be reduced. Furthermore, the color tone may be more finely adjusted.

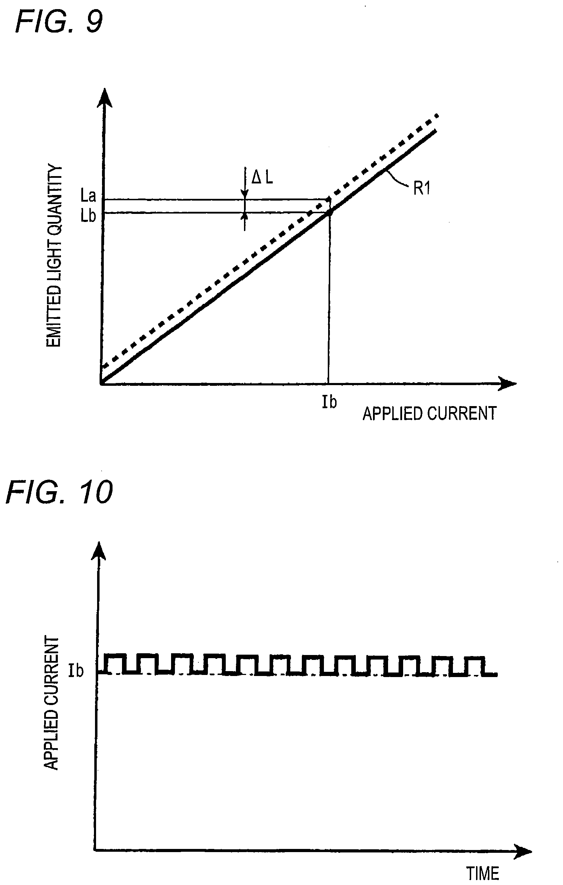

The light sources 45 and 47 may be driven as follows:
The light source control section 49 of FIG. 1 controls the output light quantity of each of the light sources 45 and 47 in accordance with an instruction issued by the control section 67. In each of the light sources 45 and 47, there is a relationship R1 between an applied current and an emitted light quantity as illustrated in FIG. 9, and hence, the applied current to each of the light sources 45 and 47 is controlled so as to attain a desired emitted light quantity. For example, in order to attain an emitted light quantity La, while securing an emitted light quantity Lb on the basis of the relationship R1 by setting the applied current to Ib, a difference ΔL between the emitted light quantities Lb and La as a fine adjustment margin is obtained by superimposing a pulse-modulated pulse current on the applied current.

As the applied current used in this case, for example, as illustrated as a pulse current superimposed waveform of the applied current in FIG. 10, the emitted light quantity La is obtained by the pulse current with the applied current Ib as bias. As pulse width control of a pulse current, an arbitrary waveform may be obtained by using an appropriate PWM circuit, and the emitted light quantity may be thus accurately set. Through such bias current control and pulse-modulated control, a settable dynamic range of the emitted light quantity may be widely secured.

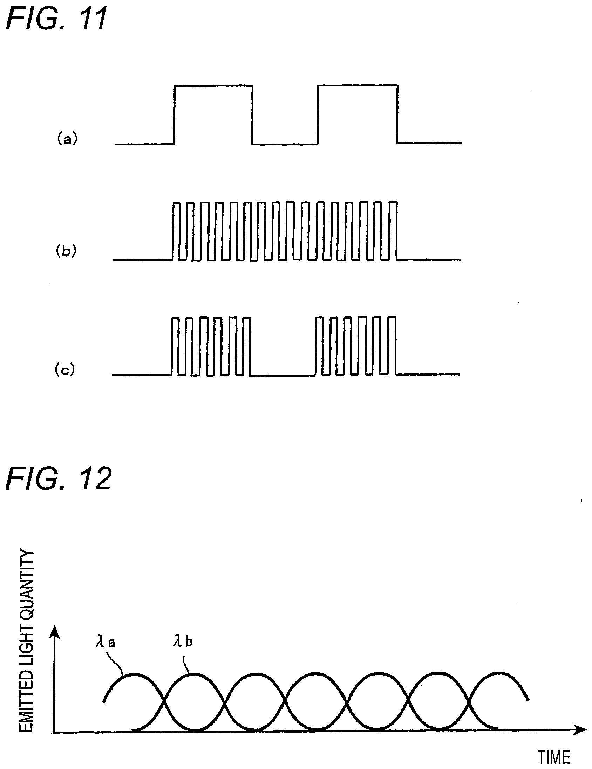

It is noted that any of various drive waveforms may be utilized for the pulse-modulated control performed in this case. For example, when a pulse waveform as illustrated in FIG. 11 (a) repeatedly turned on/off in synchronization with optical storage time per frame of an image of an imaging device is utilized, wasteful heat generation may be avoided. Alternatively, a pulse waveform as illustrated in FIG. 11 (b) with a sufficiently short cycle as compared with the optical storage time is utilized, image noise derived from speckle of laser may be reduced. Further alternatively, a pulse waveform as illustrated in FIG. 11(c) obtained by mixing the waveforms of FIG. 11(a) and FIG. 11(b) in which the on period of the pulse waveform of FIG. 11 (a) has the pulse waveform with the short cycle of FIG. 11 (b) is utilized, the aforementioned effects may be simultaneously achieved.

Furthermore, when the light sources 45 and 47 are controlled to be alternately turned on so as to alternately attain the maximum emitted light quantity as illustrated in FIG. 12, the maximum driving power of the light source device 41 for both the light sources 45 and 47 may be suppressed, so as to reduce the burden on the body of the subject. Moreover, pickup images obtained respectively with the illuminating light of the light sources 45 and 47 may be individually obtained, and in such a case, it is possible to perform an operation between the obtained images, so as to improve the degree of freedom in the image processing.

In this manner, in accordance with the observation magnifying power, that is, one of information on an access degree between the region to be observed of the subject and the endoscope tip portion 35, the ratio of the visible short wavelength component in the illuminating light is increased as the access degree is higher, so as to automatically change the color tone of the illuminating light, and thus, the illuminating light with a color tone always suitable to observation is obtained and the convenience in the operation with the endoscope is improved. Incidentally, the observation magnifying power herein means observation magnifying power on the region to be observed and is not limited to the optical magnifying power for optically changing the angle of view of an observation image by using the objective lens unit 39 as described above but may be digital zoom magnifying power for electrically changing the angle of view in the observation image obtained by the imaging device 21 (see FIG. 1). When the digital zoom magnifying power is employed, the control section 67 instructs the output light quantity ratio between the light sources 45 and 47. to the light source control section 49 on the basis of information input from the input section 17 of FIG. 1.

Furthermore, when an observation mode with different observation magnifying power is switched by operating an operation switch 117 provided on the operation section 23 of the endoscope 11, the light quantity ratio may be changed in synchronization with the operation of the operation switch 117.

### <Structure in which the color tone of the illuminating light is changed in accordance with light quantity from the region to be observed>

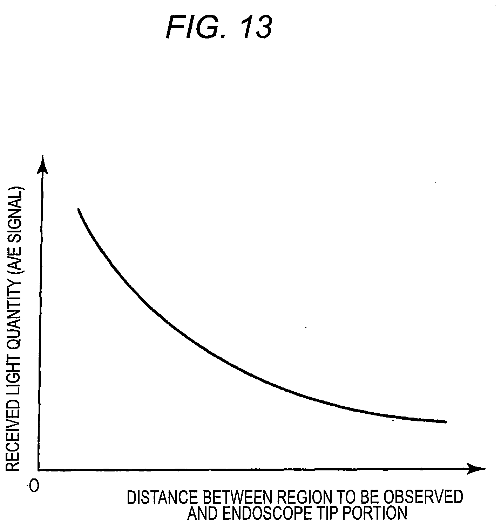

Next, as another example of the change of the color tone of the illuminating light in accordance with the information on the access degree between the region to be observed and the endoscope tip portion 35, a structure in which the color tone is changed in accordance with the quantity of light from the region to be observed on the basis of an imaging signal output from the imaging device 21 will be described. As illustrated in FIG. 13, when the distance between the region to be observed and the endoscope tip portion is large, the quantity of light received by the imaging device 21 tends to be small, and when the distance is small, the quantity of the received light tends to be large. In other words, the distance between the region to be observed and the endoscope tip portion 35 may be estimated on the basis of the quantity of light received by the imaging device 21. Therefore, a signal corresponding to the quantity of reflected light (hereinafter referred to as the A/E signal) is generated on the basis of an output signal from the imaging device 21, and the output light quantity ratio between the light sources 45 and 47 is changed on the basis of the A/E signal.

Specifically, the control section 67 determines that the distance between the region to be observed and the endoscope tip portion is smaller and the access degree is higher as the brightness of the detected A/E signal is lower, so as to make the light source control section 49 increase the ratio of the output light quantity of the violet laser light source 47 for increasing blueness in the color tone of the illuminating light. On the contrary, as the brightness of the A/E signal is higher, the ratio of the output light quantity of the blue laser light source 45 is increased, so as to control the illuminating light to be white with blueness suppressed.

When this structure is employed, the output signal from the imaging device 21 always continuously obtained during the observation with the endoscope is used for obtaining, on the basis of the A/E signal, the quantity of the reflected light, that is, one of the information on the access degree between the region to be observed of the subject and the endoscope tip portion 35, and since the distance between the region to be observed and the endoscope tip portion is thus estimated, the illuminating light may be changed to always have the optimum color tone with high responsibility.

### <Structure in which the color tone of the illuminating light is changed in accordance with a site of the region to be observed>

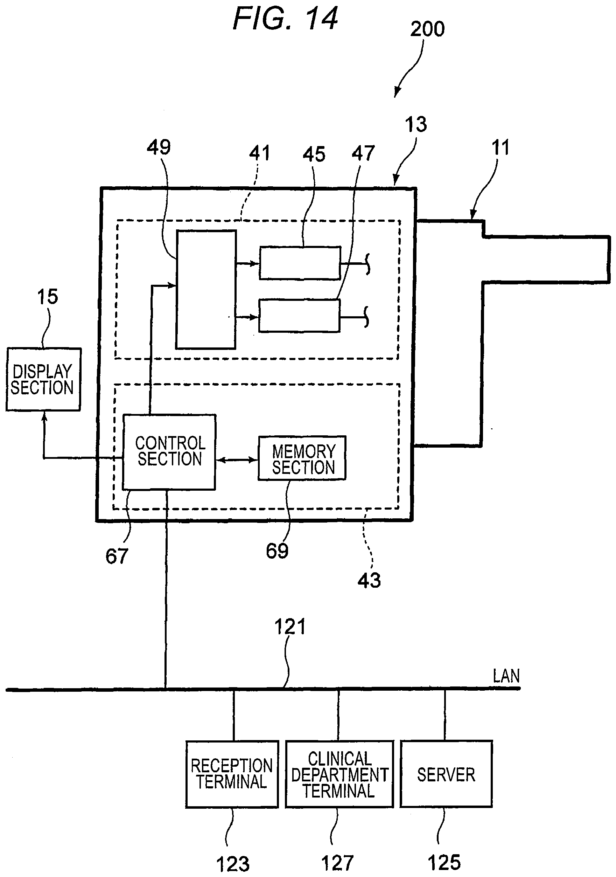

Next, an exemplary structure in which the color tone of the illuminating light is changed in accordance with a site of the region to be observed will be described. FIG. 14 is a block diagram roughly illustrating the structure of an endoscopic device employing this exemplary structure. As illustrated in FIG. 14, the endoscopic device 200 has basically the same structure as that illustrated in FIG. 1, and a controller 13 is connected to a network 121 built in a hospital. Furthermore, a reception terminal 123, a server 125, a clinical department terminal 127 and the like are connected to the network 121, so as to share various information with the endoscopic device 200.

In this exemplary structure of the endoscopic device 200, an observation site of a patient, that is, a subject, to be observed with the endoscope is extracted from test order information, and the color tone of the illuminating light is changed in accordance with information of the extracted observation site. When the color tone of the illuminating light is set to be suitable to diagnosis of the observation site, it becomes easy to find and diagnose a lesion. It is herein assumed for explanation that a patient is subjected to an endoscopic test in the hospital.

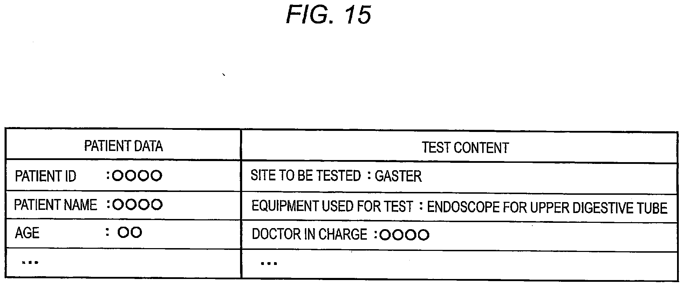

In an environment of a hospital management system such as electronic clinical recording and a test ordering system, when the patient first clears reception processing through the reception terminal 123 installed in the hospital, reception information is sent to the server 125. The server 125 refers to a memory device such as a mass storage hard disc included therein for the information of the received patient, so as to extract patient information such as a patient ID, a patient name and an age of the patient having been registered, and, if a test is scheduled, test information such as a test site for the test, equipment to be used for the test and a doctor in charge as illustrated in FIG. 15.

Thereafter, when test order information including the patient information and the test information is sent from the server 125 to the clinical department terminal 127 of a specific clinical department where the endoscopic test is to be carried out, the patient is subjected to the endoscopic test in this specific clinical department.

When the endoscopic device used in this test is the aforementioned endoscopic device 200, the control section 67 of the endoscopic device 200 acquires the information of the test site included in the test order information at prescribed timing, and sets the illuminating light to the color tone suitable to the test site. The relationship between a test site and a color tone of the illuminating light is precedently stored in the memory section 69 of the endoscopic device 200, and the control section 67 refers to the thus stored table information so as to instruct the light source control section 49 to attain a desired light quantity ratio between the light sources 45 and 47.

When this structure is employed, in the case where an observation site is known prior to the test, the color tone of the illuminating light of the endoscopic device may be set optimally to, for example, the observation site of each biological organ. For example, although the same endoscope for the upper digestive tube is used in the endoscopic test, the gaster having a large number of blood vessels has a color with strong redness as compared with the esophagus. Therefore, in observation of the gaster with the endoscope, a red component is first saturated and the quantity of green and blue light tends to be lacking in a pickup image, and hence, the S/N of an observation image is degraded. In such a case, the ratio of the blue and green components of the light sources is increased, so as to obtain an image with satisfactory S/N with respect to the three colors of red, green and blue. Incidentally, when the color tone of the light source is changed, the color tone of a pickup image is corrected for color balance through image processing, so as to constantly retain colors displayed on the display section 15 for displaying an observation image.

Incidentally, any of various setting may be employed apart from the aforementioned setting of the test site by using the hospital management system, and for example, the endoscopic device 200 may be automatically changed in the color tone of the illuminating light to be optimum to an observation site by an operator of the endoscope inputting information of the test site to the endoscopic device 200.

### <Structure in which the color tone of the illuminating light is changed in accordance with the type of endoscope>

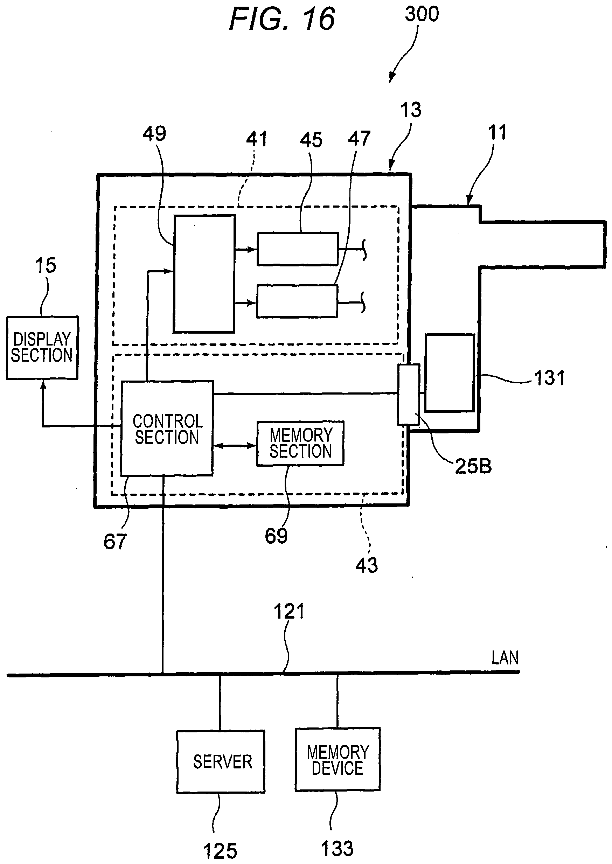

Next, an exemplary structure in which the color tone of the illuminating light is changed in accordance with the type of endoscope will be described. FIG. 16 is a block diagram roughly illustrating the structure of an endoscopic device employing this exemplary structure. As illustrated in FIG. 16, the endoscopic device 300 has basically the same structure as that illustrated in FIG. 1, and an endoscope 11 includes a main body memory section 131 storing individual identification information of the endoscope 11. Also, a controller 13 is connected to a network 121 to which a server 125 and a memory device 133 are connected.

The main body memory section 131 includes an IC memory, an IC tag or the like, and a ROM such as a flash memory or a RAM may be used as the IC memory, and an RFID (Radio frequency identification) or the like may be used as the IC tag. When the IC tag is used, a reader device may be provided in a connector section 25B, so that a signal obtained by reading the IC tag may be input to a control section 67.

In this exemplary structure of the endoscopic device 300, the individual identification information of the endoscope 11 is stored in the main body memory section 131 included in the endoscope 11, and when the endoscope 11 is connected to the controller 13 through the connector section 25B, the control section 67 reads the individual identification information from the main body memory section 131 of the endoscope 11, so as to identify the connected endoscope 11.

The control section 67 sets the color tone of the illuminating light in accordance with the individual identification information of the connected endoscope 11. The relationship between individual identification information and a color tone of the illuminating light is precedently stored in a memory section 69 of the endoscopic device 300, and the control section 67 refers to the thus stored table information, so as to instruct a light source control section 49 to attain a desired light quantity ratio between light sources 45 and 47.

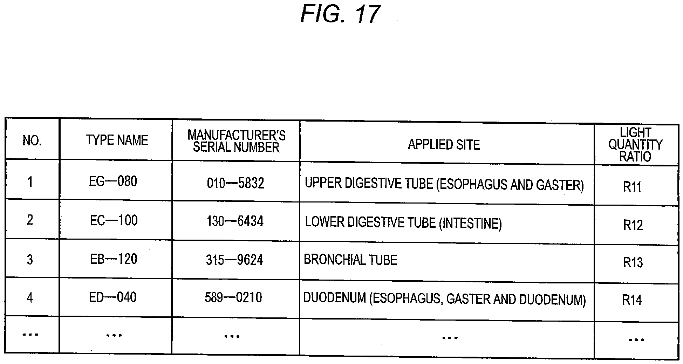

The individual identification information is, for example, the type name of the endoscope 11 different depending upon an applied site to be subjected to the test. FIG. 17 illustrates an exemplary registration table of individual identification information including the type names of endoscopes. This registration table includes individual information such as the type name, a manufacturer's serial number and an applied site of an endoscope 11 and information of a light quantity ratio between light sources for changing the color tone of the illuminating light. For example, with respect to an endoscope with the type name of "EC-100", an applied site for the test is the large intestine, and the light quantity ratio between light sources 45 and 47 (see FIG. 16) is set to R12.

When this endoscopic device 300 is used, the color tone of the illuminating light may be changed to be optimally to the endoscope 11 in accordance with the individual identification information of the endoscope 11 connected to the controller 13. It is noted that the registration table may include another parameter for specifying the color tone of the illuminating light instead of the light quantity ratio.

Furthermore, the individual identification information of the endoscope 11 may be information corresponding to a composing member of the endoscope 11 instead of the information corresponding to an applied site. For example, the individual identification information may be information on the type or component of an optical member used in the endoscope 11 such as a phosphor 57 (see FIG. 1), optical fibers 55A and 55B, a solid-state imaging device 21 or an objective lens unit 39.

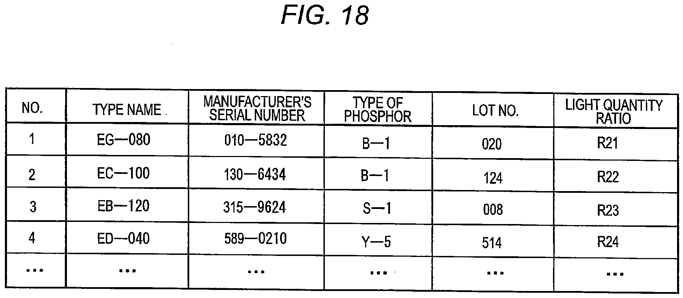

FIG. 18 illustrates an exemplary registration table of individual identification information including the type and individual specificity of a phosphor used in the endoscope 11. This registration table includes individual information such as the type name of an endoscope 11, the manufacturer's serial number, the type and a lot number of a phosphor, and information of the light quantity ratio between light sources for changing the color tone of the illuminating light. For example, an endoscope with the type name of "EG-120" is defined by the type of phosphor of "Y-5", the lot number of "514" and the light quantity ratio of R24. Since a more detailed composition of the phosphor "Y-5" may be specified by using the lot number on the basis of information obtained in fabrication by the manufacturer, a color tone delicately changed in accordance with the composition of the phosphor may be corrected to a desired color tone. In other words, it is possible to accurately define a light quantity ratio optimum to the specified phosphor.

In this manner, the color tone of the illuminating light optimum to the endoscope 11 to be used may be easily set by changing the color tone of the illuminating light in accordance with the individual identification information of the endoscope 11.

In each of the endoscopic devices 100, 200 and 300 described so far, the illuminating light is generated by multiplexing/demultiplexing the laser beams emitted by the two laser light sources 45 and 47 and guiding the resultant light to the phosphor 57, which does not limit the invention, and needless to say, another structure of the light source device may be employed. Now, other exemplary structures of the light source device will be exemplarily described.

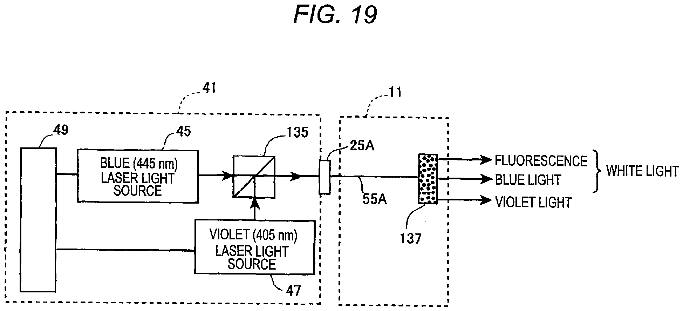

FIG. 19 is a diagram roughly illustrating the structure of an endoscopic device in which one optical fiber 55A is used as an optical path from a light source device 41 to an endoscope 11. In this structure, in the middle of an optical path where blue laser beams emitted from a blue laser light source 45 with a center wavelength of 445 nm is introduced into the optical fiber 55A, a dichroic prism 135 for merging violet laser beams emitted from a violet laser light source 47 with a center wavelength 405 nm is provided.

A phosphor 137 disposed on a light outputting side of the optical fiber 55A in this case has characteristics to absorb part of the blue laser beams emitted from the blue laser light source 45 for emitting green to yellow light through excitation, to generate white light by multiplexing the resultant light with part of the blue laser beams not absorbed but transmitted and to transmit the violet laser beams emitted from the violet laser light source 47 substantially without absorbing. Therefore, as the phosphor 137, a material for emitting light through excitation under radiation of the blue laser beams with high efficiency and generating white light by multiplexing the resultant light with the blue laser beams and a material for reducing the quantity of light emitted from the phosphor under radiation of the violet laser beams are used.

Incidentally, there is a wavelength conversion loss (a Stokes loss) such as heat generation principally caused in the wavelength conversion performed by the phosphor 137 in general. Therefore, when an excitation wavelength with a longer emission wavelength is selected, the luminous efficacy of the phosphor is higher, which is advantageous in suppression of heat generation of the phosphor. When this structure is employed, for the aforementioned reason, the white light is generated by using the laser beams of the longer wavelength, so as to improve the luminous efficacy.

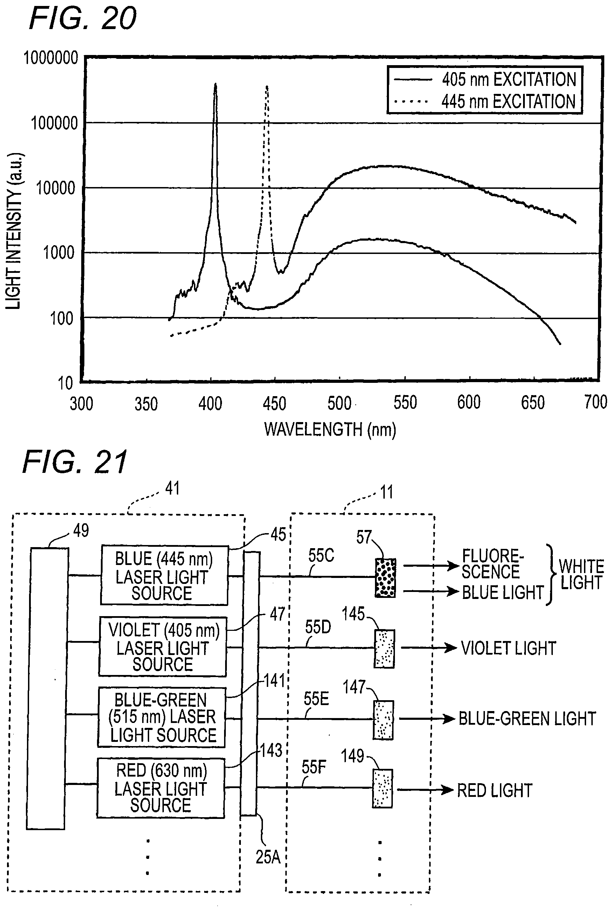

FIG. 20 illustrates examples of an emission spectrum of illuminating light obtained by the light source device 41 and the phosphor 137 of FIG. 19. As illustrated in FIG. 20, although the phosphor 137 is excited also under radiation of the violet laser beams, the quantity of light emitted from the phosphor 137 through the excitation under radiation of the violet laser beams is as small as one severalth (at least one third, preferably one fifth and more preferably one tenth or less) as compared with the quantity of light emitted through excitation under radiation of the blue laser beams. When the quantity of light emitted from the phosphor 137 through excitation under radiation of the violet laser beams is suppressed to this extent, the color temperature of the white light may be suitably retained.

When the aforementioned structure is employed, since the optical paths of the plurality of kinds of laser beams is combined by using the dichroic prism 135, the light is guided by one optical fiber 55A from the light source device 41 to the phosphor 137 of the endoscope 11 and in addition, since a radiation port of the illuminating light may be provided in one portion of the phosphor 137, the space efficiency may be improved so as to make a contribution to diameter reduction of an endoscope insert section.

Furthermore, also in the case where another laser light source is provided in addition to the blue laser light source 45 and the violet laser light source 47, the optical paths may be similarly combined through optical coupling means such as a dichroic prism. Also, the phosphor 137 may be made of a fluorescent material that is not or minimally excited under radiation of light of the wavelength of the additional laser light source.

At this point, as a specific material for the phosphor 137 to be employed in the aforementioned exemplified structure, for example, a crystalline solid-state fluorescent material including lead (Pb) as an additional element and digallium calcium tetrasulfide (CaGa₂S₄) as a parent body as described in JP-A-2006-2115, or a crystalline solid-state fluorescent material including lead (Pb) and cerium (Ce) as additional elements and digallium calcium tetrasulfide (CaGa₂S₄) as a parent body may be used. When such a fluorescent material is used, fluorescence covering substantially the whole visible range of approximately 460 nm through approximately 660 nm may be obtained, so as to improve color rendering in radiation with the white light.

Alternatively, a combination of a green phosphor of LiTbW₂O₈ (see "Phosphor for White light emitting diode", Tsutomu Odaki, Technical Report ED2005-28 of Institute of Electronics, Information and Communication Engineers, CFM2005-20, SDM2005-28, pp. 69-74 (2005-05) or the like), a β-salon (Eu) blue phosphor (see "New Sialon phosphors and white LEDs", Naoto Hirosaki, Rong-Jun Xie and Ken Sakuma, Oyo Buturi Vol. 74, No. 11, pp. 1449-1452 (2005) or Akira Yamamoto, Tokyo University of Technology, School of Bionics, Oyo Buturi, Vol. 76, No. 3, p. 241 (2007)), a CaAlSiN₃ red phosphor and the like may be used. The β-sialon is a crystal including aluminum and an acid solid solved in a β-silicon nitride crystal and having a composition of Si_{6-z}Al₂O₂N_{8-z} (wherein z is the ratio of a solid solved component). In the phosphor 137, the LiTbW₂O₈, the β-salon and CaAlSiN₃ may be mixedly present or layers of these phosphors may be stacked.

FIG. 21 is a diagram roughly illustrating the structure of another endoscopic device using a large number of laser light sources. When the structure illustrated in FIG. 21 is employed, blue laser beams emitted from a blue laser light source 45 with a center wavelength of, for example, 445 nm are guided through a connector section 25A to an optical fiber 55C included in an endoscope 11, so as to radiate a phosphor 57 disposed at a light outputting end of the optical fiber 55C. Therefore, light emitted through excitation from the phosphor 57 under radiation of excitation light of the blue laser beams and part of the blue laser beams transmitting the phosphor 57 together generate white light.

Furthermore, laser beams respectively emitted from a violet laser light source 47 with a center wavelength of, for example, 405 nm, a blue-green laser light source 141 with a center wavelength of, for example, 515 nm and a red laser light source 143 with a center wavelength of, for example, 630 nm are respectively introduced through the connector section 25A to optical fibers 55D, 55E and 55F, so as to give violet, blue-green and red illuminating light respectively through light deflecting/diffusing members 145, 147 and 149.

Each of the light deflecting/diffusing members 145, 147 and 149 may be made of any material that may transmit incident laser beams, and for example, a resin material with a translucent property, glass or the like is used. Alternatively, each of the light deflecting/diffusing members 145, 147 and 149 may be made of a resin material or glass having, on its surface, a light diffusing layer with fine irregularities or particles (fillers or the like) with different refractive indexes mixedly provided, or a semitransparent material. In this manner, transmitted light output from each of the light deflecting/diffusion members 145, 147 and 149 is obtained as illuminating light of a narrowed band wavelength in which light quantity is made uniform within a prescribed radiation region.

When a large number of laser light sources with different emission wavelengths are thus used for generating the illuminating light by adjusting the light quantity ratio among the respective light sources, the range of the color tone that may be generated by a light source control section 49 may be increased, and the adjustment range of the color tone suitable to observation may be expanded. Furthermore, delicate adjustment of the color tone may be easily and accurately carried out.

Incidentally, it is preferable to selectively use an optimum fiber in accordance with the wavelength to be employed as each of the optical fibers 55C, 55D, 55E and 55F. The core of an optical fiber has wavelength dependency with a transmission loss changed in accordance with the level of a hydroxyl group (OH⁻) density, and exhibits different absorptance against a specific infrared wavelength from that against a wavelength of the visible range. Therefore, when the wavelength of the light source is not more than 650 nm, an optical fiber including a core with a high hydroxyl group density is used, and when the wavelength exceeds 650 nm, an optical fiber including a core with a low hydroxyl group density is used.

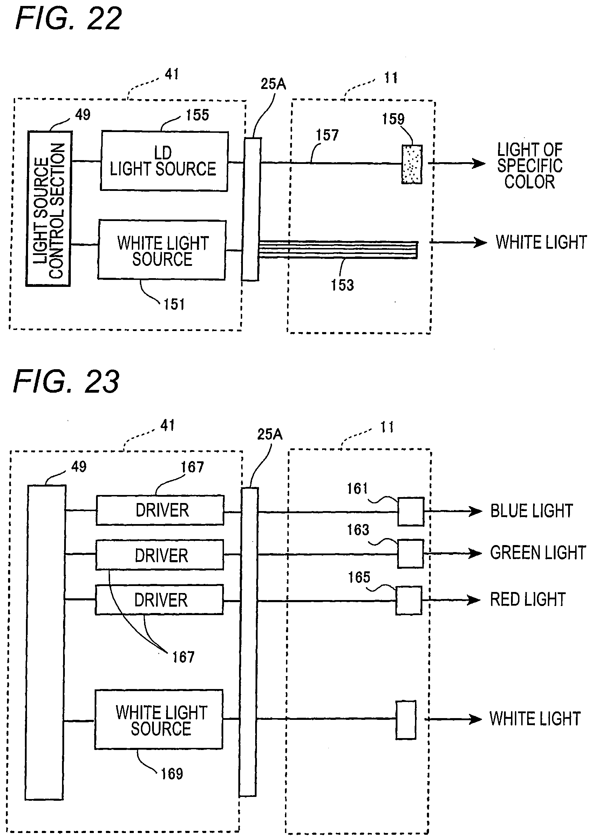

FIG. 22 is a diagram roughly illustrating the structure of another exemplary endoscopic device using a white light source and a laser light source. In this exemplary structure, a light source for emitting light of a broad wavelength band, such as a halogen lamp, a xenon lamp or a white light emitting diode, is used as the white light source 151, so as to radiate white light from a tip portion of an endoscope 11 through a light guide 153, that is, an optical fiber bundle. The color tone of the illuminating light is changed in accordance with the output light quantity of one or plural kinds of the laser light source 155. The output light from the laser light source 155 is transferred through a connector section 25 by an optical fiber 157 to the endoscope tip portion so as to be emitted as illuminating light of a specific color from a light deflecting/diffusing member 159 disposed at the light outputting end of the optical fiber 157. Alternatively, the light deflecting/diffusing member 159 may be replaced with a phosphor so as to emit fluorescence of a desired color as the illuminating light.

When the aforementioned structure is employed, the color tone of the illuminating light may be changed with a simple structure.

FIG. 23 is a diagram roughly illustrating the structure of an endoscopic device including a light emitting diode disposed in a tip portion of an endoscope 11. In this exemplary structure, the light emitting diode is provided in the tip portion of the endoscope 11. As the light emitting diode, for example, a blue light emitting diode 161, a green light emitting diode 163 and a red light emitting diode 165 are used, so as to be individually controlled for the emitted light quantity by drivers 167 connected to a light control section 49 of a light source device 41.

When this structure is employed, illuminating light of an arbitrary color tone including white may be obtained by using light emitting devices of the primary colors of light. Alternatively, a white light source 169 may be additionally provided for increasing the light quantity of white light so as to supply the white light to the endoscope 11. As the white light source 169 used in this case, a halogen lamp, a xenon lamp, a white light emitting diode or the aforementioned light source obtained by combining a laser light source and a phosphor may be used.

Although the color tone of the illuminating light is controlled by adjusting the output light quantity of each light source by the light source control section 49 in each of the aforementioned structures of the endoscopic device, the color tone may be changed without changing the output light quantity of each light source. For example, when the respective light sources are individually turned on and exposure time of the imaging device 21 at on timings of each light source is changed, the color tone of the illuminating light is changed in a pseudo manner. Specifically, the respective light sources and the imaging device 21 are synchronously controlled, so that exposure times such as exposure time of the imaging device 21 for obtaining a pickup image with merely a first light source of, for example, a blue laser light source turned on and exposure time for obtaining a pickup image with merely a second light source of, for example, a white light source turned on may be individually adjusted to be increased/decreased, and the thus obtained pickup images are synthesized to give observation image data. In this observation image data, the exposure time with each light source turned on corresponds to the light quantity of the light source, and hence, the color tone of the illuminating light may be regarded to be changed in a pseudo manner. In other words, instead of directly controlling the color tone of the illuminating light in accordance with the output light quantities of the respective light sources, the color tone of the illuminating light may be indirectly changed by individually adjusting the exposure time of the imaging device 21 with each illuminating light by using imaging controlling means for synchronously controlling the light sources and the imaging device.

In this manner, the present invention is not limited to the aforementioned embodiment but it is to be understood that changes and modifications may be made by those skilled in the art on the basis of the description and the known techniques, and the appended claims are intended to cover all such changes and modifications.

The followings are herein disclosed:
(1) According to an aspect of the invention, an endoscopic device comprises:
   an illuminating unit which emits illuminating light from an endoscope tip portion, and includes a plurality of kinds of light sources different from one another in an emission wavelength;
   an imaging unit which obtains a pickup image of a region to be observed of a subject radiated with the illuminating light; and
   the color tone controlling unit which changes a color tone of the illuminating light by changing an output light quantity ratio among the plurality of kinds of light sources.

   According to this endoscopic device, the color tone of the illuminating light may be arbitrarily changed in accordance with an observation target or any of various diagnosis situations such as enlargement observation, and hence, the observation may be performed by using the illuminating light of an optimum color tone.
(2) In the endoscopic device according to (1), the color tone controlling unit continuously changes the color tone of the illuminating light.
   According to this endoscopic device, the color tone may be delicately adjusted, and hence, a desired color tone of the illuminating light may be accurately obtained.
(3) In the endoscopic device according to (1) or (2), at least one of the plurality of kinds of light sources is a light source providing a semiconductor light emitting diode as a light emitting source.
   According to this endoscopic device, the color tone may be controlled with high resolution, the color tone is minimally changed with time, and the light quantity may be control with high responsibility. Furthermore, since high luminous efficacy is attained, the power consumption is reduced.
(4) In the endoscopic device according to any one of (1) through (3), at least one of the plurality of kinds of light sources is a white light source.
   According to this endoscopic device, since light emitted from a light source of another specific color is distributed in white illuminating light emitted from the white light source, the color tone may be easily changed with a sufficient light quantity secured.
(5) In the endoscopic device according to (4), the white light source includes:
   an excitation light source providing a semiconductor light emitting diode as a light emitting source; and
   a phosphor excited for emitting light under radiation of output light of the excitation light source.

   According to this endoscopic device, output light emitted from the excitation light source of a specific narrowed wavelength band and light emitted from the phosphor through excitation under radiation of the output light are used for obtaining illuminating light with a wide wavelength band.
(6) In the endoscopic device according to any one of (1) through (5), the color tone controlling unit controls the color tone on the basis of access degree information on an access degree between the region to be observed of the subject and the endoscope tip portion.
   According to this endoscopic device, the color tone is controlled in accordance with the access degree information corresponding to a distance between the region to be observed and the endoscope tip portion, and hence, the illuminating light may be substantially optimized for each observation target or each diagnosis situation.
(7) In the endoscopic device according to (6), the color tone controlling unit increases a ratio of a visible short wavelength component included in the pickup image as the access degree of the access degree information is higher.
   According to this endoscopic device, the ratio of the visible short wavelength component is increased as the access degree is higher, namely, as the distance between the region to be observed and the endoscope tip portion is shorter, and hence, for example, when a blood vessel of a surface layer is desired to observe, blood vessel information may be highlighted in an image.
(8) In the endoscopic device according to (6) and (7), the access degree information is magnifying power information corresponding to observation magnifying power for the region to be observed.
   According to this endoscopic device, the information on the observation magnifying power associated with the level of the access degree between the region to be observed and the endoscope tip portion is used, so as to change the color tone suitably to the access degree, and hence, observation substantially optimum to the observation target or the diagnosis situation may be performed.
(9) In the endoscopic device according to (8), the magnifying power information is information on optical magnifying power of an imaging optical system that optically changes an angle of view of an observation image obtained by the imaging unit.
   According to this endoscopic device, since the color tone is changed in synchronization with an operation performed by an operator of the endoscope or the like for changing the optical magnifying power, the color tone may be rapidly changed on a real-time basis.
(10) In the endoscopic device according to (8), the magnifying power information is information on digital zoom magnifying power for electrically changing an angle of view of an observation image obtained by the imaging unit.
   According to this endoscopic device, since the color tone is changed in synchronization with an operation performed by an operator of the endoscope or the like for changing the digital zoom magnifying power, the color tone may be rapidly changed on a real-time basis.
(11) In the endoscopic device according to (6) or (7), the access degree information is light quantity information of the pickup image obtained by the imaging unit.
   According to this endoscopic device, since the color tone is changed by utilizing the light quantity information always obtained continuously during the observation with the endoscope and output from the imaging device, the color tone may be always optimally adjusted with high responsibility.
(12) In the endoscopic device according to (11), the color tone controlling unit determines that the access degree is higher as a value of a light quantity of the light quantity information is smaller.
   According to this endoscopic device, the access degree may be estimated by utilizing a fact that the quantity of light received by the imaging device is smaller as the distance between the region to be observed and the endoscope tip portion is larger.
(13) In the endoscopic device according to any one of (1) through (5), the color tone controlling unit controls the color tone on the basis of information on an observation site of the subject.
   According to this endoscopic device, the color tone is adjusted to be suitable to diagnosis of the observation site, so as to easily find and diagnose a lesion.
(14) In the endoscopic device according to (13), the information on the observation site is information defined with respect to each biological organ of the subject.
   According to this endoscopic device, the color tone may be adjusted to be optimum for diagnosis of each biological organ.
(15) The endoscopic device according to any one of (1) through (5) further may comprise:
   an endoscope main body which includes the endoscope tip portion;
   an endoscope control section which is connected to the endoscope main body; and
   a main body memory section which stores individual identification information of the endoscope main body in the endoscope main body, wherein
   the color tone controlling unit controls the color tone on the basis of the individual identification information stored in the main body memory section.

   According to this endoscopic device, the color tone may be optimally adjusted in accordance with the individual identification information of the endoscope main body.
(16) In the endoscopic device according to (15), the individual identification information includes type information including information on an applied site of the subject to which the endoscope main body is applicable, and
   the color tone controlling unit controls the color tone in accordance with the type information.
   According to this endoscopic device, the observation may be performed with the color tone suitable to the applied site.
(17) In the endoscopic device according to (16), a phosphor excited for emitting light under radiation of excitation light emitted from at least one of the plurality of kinds of light sources is provided in the endoscope main body,
   individual information corresponding to a composition of the phosphor is included in the individual identification information, and
   the color tone controlling unit controls the color tone in accordance with the individual information of the phosphor.

According to this endoscopic device, the color tone varying depending upon the composition of the phosphor may be accurately corrected to a desired color tone.

## Claims

1. An endoscopic device comprising:
an illuminating unit which emits illuminating light from an endoscope tip portion, and includes a plurality of kinds of light sources different from one another in an emission wavelength;
an imaging unit which obtains a pickup image of a region to be observed of a subject radiated with the illuminating light; and
the color tone controlling unit which changes a color tone of the illuminating light by changing an output light quantity ratio among the plurality of kinds of light sources,
**characterized in that** the color tone controlling unit (67) is adapted to control the color tone on the basis of access degree information on an access degree between the region to be observed of the subject and the endoscope tip portion (35), wherein the access degree being dependent on e.g. the observation magnifying power on the region to be observed or the quantity of light reflected from that region.

2. The endoscopic device according to claim 1,
wherein the color tone controlling unit continuously changes the color tone of the illuminating light.

3. The endoscopic device according to claim 1 or 2,
wherein at least one of the plurality of kinds of light sources is a light source providing a semiconductor light emitting diode as a light emitting source.

4. The endoscopic device according to any of claims 1 through 3,
wherein at least one of the plurality of kinds of light sources is a white light source.

5. The endoscopic device according to claim 4,
wherein the white light source includes:
an excitation light source providing a semiconductor light emitting diode as a light emitting source; and
a phosphor excited for emitting light under radiation of output light of the excitation light source.

6. The endoscopic device according to claim 1,
wherein the color tone controlling unit increases a ratio of a visible short wavelength component included in the pickup image as the access degree of the access degree information is higher.

7. The endoscopic device according to any of claims 1 to 6,
wherein the access degree information is magnifying power information corresponding to observation magnifying power for the region to be observed.

8. The endoscopic device according to claim 7,
wherein the magnifying power information is information on optical magnifying power of an imaging optical system that optically changes an angle of view of an observation image obtained by the imaging unit.

9. The endoscopic device ccording to claim 7,
wherein the magnifying power information is information on digital zoom magnifying power for electrically changing an angle of view of an observation image obtained by the imaging unit.

10. The endoscopic device according to any of claims 1 to 6,
wherein the access degree information is light quantity information of the pickup image obtained by the imaging unit.

11. The endoscopic device according to claim 10,
wherein the color tone controlling unit determines that the access degree is higher as a value of a light quantity of the light quantity information is smaller.

12. The endoscopic device according to any of claims 1 through 5,
wherein the color tone controlling unit controls the color tone on the basis of information on an observation site of the subject.

13. The endoscopic device according to claim 12,
wherein the information on the observation site is information defined with respect to each biological organ of the subject.

14. The endoscopic device according to any of claims 1 through 5, further comprising:
an endoscope main body which includes the endoscope tip portion;
an endoscope control section which is connected to the endoscope main body; and
a main body memory section which stores individual identification information of the endoscope main body in the endoscope main body, wherein
the color tone controlling unit controls the color tone on the basis of the individual identification information stored in the main body memory section.

15. The endoscopic device according to claim 14,
wherein the individual identification information includes type information including information on an applied site of the subject to which the endoscope main body is applicable, and
the color tone controlling unit controls the color tone in accordance with the type information.

16. The endoscopic device according to claim 15,
wherein a phosphor excited for emitting light under radiation of excitation light emitted from at least one of the plurality of kinds of light sources is provided in the endoscope main body,
individual information corresponding to a composition of the phosphor is included in the individual identification information, and
the color tone controlling unit controls the color tone in accordance with the individual information of the phosphor.

## Patentansprüche

1. Endoskopgerät, umfassend:
eine Beleuchtungseinheit, die Beleuchtungslicht aus einem Endoskop-Endteil emittiert, und die mehrere Arten von Lichtquellen enthält, die voneinander in einer Emissionswellenlänge verschieden sind;
eine Bildgebungseinheit, die ein Aufnahmebild einer mit dem Beleuchtungslicht bestrahlten, zu betrachtenden Zone eines Subjekts aufnimmt; und
eine Farbtonsteuereinheit, die einen Farbton des Beleuchtungslichts ändert durch Ändern eines Ausgangslicht-Mengenverhältnisses unter den mehreren Arten von Lichtquellen,
**dadurch gekennzeichnet, dass** die Farbtonsteuereinheit (67) dazu ausgebildet ist, den Farbton auf der Grundlage von Zugangsmaßinformation bezüglich eines Zugangsmaßes zwischen der zu betrachtenden Zone des Subjekts und dem Endoskop-Endteil (35) zu steuern, wobei das Zugangsmaß abhängt von beispielsweise der Betrachtungs-Vergrößerung an der zu betrachtenden Zone oder der von der Zone reflektierten Lichtmenge.

2. Endoskopgerät nach Anspruch 1,
bei dem die Farbtonsteuereinheit den Farbton des Beleuchtungslichts kontinuierlich ändert.

3. Endoskopgerät nach Anspruch 1 oder 2,
bei dem mindestens eine der mehreren Arten von Lichtquelle eine Lichtquelle ist, die als Licht emittierende Quelle eine Halbleiter-Leuchtdiode bereitstellt.

4. Endoskopgerät nach einem der Ansprüche 1 bis 3,
bei dem mindestens eine der mehreren Arten von Lichtquellen eine Weißlichtquelle ist.

5. Endoskopgerät nach Anspruch 4,
bei dem die Weißlichtquelle enthält:
eine Anregungslichtquelle, die eine Halbleiter-Leuchtdiode als Licht emittierende Quelle bereitstellt; und
einen Leuchtstoff, der zum Emittieren von Licht angeregt wird unter Bestrahlung von Ausgangslicht der Anregungslichtquelle.

6. Endoskopgerät nach Anspruch 1,
bei dem die Farbtonsteuereinheit ein Verhältnis einer sichtbaren kurzwelligen Komponente in dem Aufnahmebild erhöht, wenn das Zugangsmaß der Zugangsmaßinformation größer ist.

7. Endoskopgerät nach einem der Ansprüche 1 bis 6,
bei dem die Zugangsmaßinformation eine Vergrößerung entsprechend einer Betrachtungsvergrößerung für die zu betrachtende Zone ist.

8. Endoskopgerät nach Anspruch 7,
bei dem die Vergrößerungsinformation Information über eine optische Vergrößerung einer Abbildungsoptik ist, welche einen Betrachtungswinkel eines von der Bildgebungseinheit erhaltenen Betrachtungsbilds optisch ändert.

9. Endoskopgerät nach Anspruch 7, bei dem die Vergrößerungsinformation Information über eine digitale Zoom-Vergrößerung zum elektrischen Ändern eines Gesichtswinkels eines von der Bildgebungseinheit erhaltenen Betrachtungsbilds ist.

10. Endoskopgerät nach einem der Ansprüche 1 bis 6, bei dem die Zugangsmaßinformation Lichtmengeninformation des von der Bildgebungseinheit erhaltenen Aufnahmebilds ist.

11. Endoskopgerät nach Anspruch 10, bei dem die Farbtonsteuereinheit feststellt, dass das Zugangsmaß höher ist, wenn ein Wert einer Lichtmenge der Lichtmengeninformation kleiner ist.

12. Endoskopgerät nach einem der Ansprüche 1 bis 5, bei dem die Farbtonsteuereinheit den Farbton auf der Grundlage der Information über einen Betrachtungsort des Subjekts steuert.

13. Endoskopgerät nach Anspruch 12, bei dem die Information über den Betrachtungsort Information ist, die in Bezug auf jedes biologische Organ des Subjekts definiert ist.

14. Endoskopgerät nach einem der Ansprüche 1 bis 5, weiterhin umfassend:
einen Endoskophauptkörper, der das Endoskop-Endteil enthält;
einen Endoskopsteuerabschnitt, der an den Endoskophauptkörper angeschlossen ist; und
einen Hauptkörper-Speicherabschnitt, der individuelle Kennungsinformation des Endoskophauptkörpers in diesem speichert, wobei
die Farbtonsteuereinheit den Farbton auf der Grundlage der in dem Hauptkörperspeicherabschnitt gespeicherten individuellen Kennungsinformation steuert.

15. Endoskopgerät nach Anspruch 14, bei dem die individuelle Kennungsinformation Typeninformation enthält, darunter Information über einen Anwendungsort des Subjekts, bei dem der Endoskophauptkörper einsetzbar ist, und
die Farbtonsteuereinheit den Farbton nach Maßgabe der Typeninformatoin steuert.

16. Endoskopgerät nach Anspruch 15,
bei dem in dem Endoskophauptkörper ein Leuchtstoff vorgesehen ist, der zum Emittieren von Licht angeregt wird unter Bestrahlung von Anregungslicht, das von mindestens einer der mehreren Arten von Lichtquellen emittiert wird,
individuelle Information entsprechend einer Zusammensetzung des Leuchtstoffs in der individuellen Kennungsinformation enthalten ist, und
die Farbtonsteuereinheit den Farbton abhängig von der individuellen Information über den Leuchtstoff steuert.

## Revendications

1. Dispositif endosçopique, comprenant :
une unité d'éclairage, laquelle émet une lumière d'éclairage à partir d'une portion de bout d'endoscope, et inclut une pluralité de types de sources lumineuses différentes entre elles quant à une longueur d'onde d'émission ;
une unité d'imagerie, laquelle obtient une image de prise de vue d'une région à observer d'un sujet irradié avec la lumière d'éclairage, et
une unité de commande de tonalité de couleur, laquelle modifie une tonalité de couleur de la lumière d'éclairage en modifiant un rapport de quantité de lumière de sortie parmi la pluralité de types de sources lumineuses,
**caractérisé en ce que** l'unité de commande de tonalité de couleur (67) est apte à commander la tonalité de couleur sur la base d'informations de degré d'accès sur un degré d'accès entre la région à observer du sujet et la portion de bout d'endoscope (35), dans lequel le degré d'accès dépend par exemple de la puissance de grossissement d'observation sur la région à observer ou de la quantité de lumière réfléchie à partir de ladite région.

2. Dispositif endoscopique selon la revendication 1,
dans lequel l'unité de commande de tonalité de couleur modifie en continu la tonalité de couleur de la lumière d'éclairage.

3. Dispositif endoscopique selon la revendication 1 ou 2,
dans lequel au moins une source de la pluralité de types de sources lumineuses est une source lumineuse fournissant une diode électroluminescente à semi-conducteur comme source d'émission lumineuse.

4. Dispositif endoscopique selon l'une quelconque des revendications 1 à 3,
dans lequel au moins une source de la pluralité de types de sources lumineuses est une source de lumière blanche.

5. Dispositif endoscopique selon la revendication 4,
dans lequel la source de lumière blanche inclut :
une source lumineuse d'excitation fournissant une diode électroluminescente à semi-conducteur comme source d'émission lumineuse, et
un luminophore excité pour émettre une lumière sous le rayonnement d'une lumière de sortie de la source lumineuse d'excitation.

6. Dispositif endoscopique selon la revendication 1,
dans lequel l'unité de commande de tonalité de couleur augmente un rapport d'une composante de longueur d'onde courte visible incluse dans l'image de prise de vue à mesure que le degré d'accès des informations de degré d'accès devient plus élevé.

7. Dispositif endoscopique selon l'une quelconque des revendications 1 à 6,
dans lequel les informations de degré d'accès sont des informations de puissance de grossissement correspondant à une puissance de grossissement d'observation pour la région à observer.

8. Dispositif endoscopique selon la revendication 7,
dans lequel les informations de puissance de grossissement sont des informations sur une puissance de grossissement optique d'un système optique d'imagerie qui modifie par voie optique un angle de vue d'une image d'observation obtenue par l'unité d'imagerie.

9. Dispositif endoscopique selon la revendication 7,
dans lequel les informations de puissance de grossissement sont des informations sur une puissance de grossissement de zoom numérique pour modifier par voie électrique un angle de vue d'une image d'observation obtenue par l'unité d'imagerie.

10. Dispositif endoscopique selon l'une quelconque des revendications 1 à 6,
dans lequel les informations de degré d'accès sont des informations de quantité lumineuse de l'image de prise de vue obtenue par l'unité d'imagerie.

11. Dispositif endoscopique selon la revendication 10,
dans lequel l'unité de commande de tonalité de couleur détermine que le degré d'accès devient plus élevé à mesure qu'une valeur d'une quantité lumineuse des informations de quantité lumineuse devient plus petite.

12. Dispositif endoscopique selon l'une quelconque des revendications 1 à 5,
dans lequel l'unité de commande de tonalité de couleur commande la tonalité de couleur sur la base d'informations sur un site d'observation du sujet.

13. Dispositif endoscopique selon la revendication 12,
dans lequel les informations sur le site d'observation sont des informations définies par rapport à chaque organe biologique du sujet.

14. Dispositif endoscopique selon l'une quelconque des revendications 1 à 5, comprenant en outre :
un corps principal d'endoscope, lequel inclut la portion de bout d'endoscope ;
une section de commande d'endoscope, laquelle est connectée au corps principal d'endoscope, et
une section de mémoire de corps principal, laquelle stocke des informations d'identification individuelles du corps principal d'endoscope dans le corps principal d'endoscope,
dans lequel
l'unité de commande de tonalité de couleur commande la tonalité de couleur sur la base d'informations d'identification individuelles stockées dans la section de mémoire de corps principal.

15. Dispositif endoscopique selon la revendication 14,
dans lequel les informations d'identification individuelles incluent des informations de type incluant des informations sur un site appliqué du sujet sur lequel le corps principal d'endoscope peut être appliqué, et
l'unité de commande de tonalité de couleur commande la tonalité de couleur en fonction des informations de type.

16. Dispositif endoscopique selon la revendication 15,
dans lequel un luminophore excité pour émettre une lumière sous l'irradiation d'une lumière d'excitation émise à partir d'au moins une source parmi la pluralité de types de sources lumineuses est fourni dans le corps principal d'endoscope ;
des informations individuelles correspondant à une composition du luminophore sont incluses dans les informations d'identification individuelles, et
l'unité de commande de tonalité de couleur commande la tonalité de couleur en fonction des informations individuelles du luminophore.
